# EUROPÄISCHE PATENTANMELDUNG

(11) **EP 1 138 374 A1**
(43) Veröffentlichungstag der Anmeldung: **04.10.2001**
(21) Anmeldenummer: 00106710.7
(22) Anmeldetag: 29.03.2000
(51) Int. Cl.: B01F 15/04, B44D 3/00, A45D 44/00

(54) **Verfahren zur Herstellung von Haarfarben oder Tönungen**

(71) Anmelder: Birner, Günther, 82131 Gauting (DE)
(72) Erfinder: Koehler, Joachim, Dr., 64395 Brensbach (DE); Birner, Günther, 82131 Gauting (DE)
(74) Vertreter: Grünecker, Kinkeldey, Stockmair & Schwanhäusser Anwaltssozietät

(57) **Zusammenfassung**

Verfahren zur maschinellen, individuellen Vorortherstellung von gebrauchsfertigen Haarfarben oder Tönungen, das folgende Schritte aufweist: Bestimmen des von einem Kunden gewünschten Farbtons und der benötigten Farbmenge der herzustellenden Haarfarbe oder Tönung; separates Bereitstellen einer Vielzahl von für die Herstellung von Haarfarben oder Tönungen benötigten Farbkupplern, weiteren Grundkomponenten wie beispielsweise Oxidations-Basen, Alkalisierungsmitteln, Emulgatoren, Stabilisatoren und oberflächenaktiven Mitteln, sowie mindestens eines Oxidationsmittels; Bereitstellen von für unterschiedliche Haarfarben oder Tönungen benötigten Mischungsverhältnissen aus Farbkupplern, weiteren Grundkomponenten sowie Oxidationsmittel mittels eines Speichers; Auslesen des zur gewünschten Haarfarbe korrespondierenden Mischungsverhältnisses aus dem Speicher; Bestimmen der benötigten Dosierungsvolumina anhand des Mischungsverhältnisses und der benötigten Farbmenge; maschinelles Zuführen und Vermischen der entsprechenden Dosierungsvolumina aus Farbkupplern, weiteren Grundkomponenten und Oxidationsmittel, und Ausgeben der benötigten hergestellten Menge der Haarfarbe oder Tönung zur Applikation am Kunden.

## Beschreibung

Die vorliegende Erfindung bezieht sich auf ein Verfahren zur maschinellen, individuellen Vorortherstellung von gebrauchsfertigen Haarfarben oder Tönungen gemäß dem Gegenstand des Patentanspruchs 1 sowie auf eine Vorrichtung zur Herstellung von Haarfarben oder Tönungen gemäß dem Oberbegriff des Patentanspruchs 12.

Bei der Haarbehandlung wird das Kopfhaar einer Person entweder individuell gepflegt, d. h. von der Person selbst, oder professionell behandelt, d. h. von einem Frisör. Hierbei bietet der Frisör vielfältige Behandlungsmöglichkeiten an, wie beispielsweise Waschen, Schneiden, Tönen, Färben oder Erstellen einer Dauerwelle.

In der Regel verwendet der Frisör spezifische Haarbehandlungsmittel, die in Fabriken hergestellt und in Varianten der Zusammensetzung, wie auch der Verpackung, vertrieben werden. Gebräuchliche Haarbehandlungsmittel sind Shampoos, Haarpflegemittel, wie beispielsweise Haarspülungen und Kurpackungen, Haarfestiger, Haarverformungsmittel, wie beispielsweise Dauerwellen-Lösungen, Tönungen und Haarfarben, wobei es sich in der Regel um flüssige, gelige, cremige oder Aerosol-Produkte handelt. Im Bereich Shampoo werden ungefähr 4 bis 6 verschiedene Ausrichtungen, bei Dauerwellen 3 bis 4 verschiedene Ausrichtungen, bei Festigern ebenso 3 bis 4 verschiedene Ausrichtungen, bei Tönungen ungefähr 6 bis 10 verschiedene Ausrichtungen und bei Haarfarben ungefähr 40 bis 60 unterschiedliche Töne angeboten. Insbesondere haben sich bei den Verpackungen auch Mengenstandards entwickelt, die zumeist auf eine maximal benötigte Menge des entsprechenden Haarbehandlungsmittels ausgerichtet sind.

Von den aufgeführten Haarbehandlungsmitteln bilden insbesondere die Tönungen und Haarfarben ein wirtschaftlich sehr bedeutendes Marktsegment, das in den letzten zehn Jahren alljährlich deutliche Zuwächse verzeichnet. Da dieser Trend sich vorerst fortzusetzen scheint, wird versucht, bezüglich zur Verfügung stehender Farbabstimmungen möglichst eine Abhängigkeit zwischen einem Frisör bzw. einem Kunden und Herstellern von Tönungen und Haarfarben zu erzielen. Somit läßt sich erklären, daß einige wenige Hersteller momentan fast vollständig dieses Marktsegment beherrschen. So findet man hinsichtlich Tönungen beispielsweise die Firmen Henkel, Wella, L'Oréal und Schwarzkopf in einer marktdominierenden Position. Bei Haarfarben sind wiederum die Firmen Wella, L'Oréal und Schwarzkopf führend, wobei zusätzlich die Firma Goldwell hinzukommt.

Die Haartönung ist eine Färbung der Haare, die durch sog. direktziehende Farbstoffe ausgeführt wird, wobei diese Farbstoffe durch Waschen der Haare wieder entfernt werden. Haartönungen sind heutzutage als flüssige Tönungsfarbe oder als Tönungsschaum erhältlich, aber auch Tönungs-Cremes und -Shampoos sind marktüblich. Gebräuchliche Haartönungen überdauern in etwa drei bis acht Haarwäschen und werden sehr stark individuell genutzt, d.h. im Heimbereich der betreffenden Personen. Hierzu bietet jeder der oben genannten Firmen in der Regel eine Farbpalette von ca. zehn bis zwanzig unterschiedlichen Farben an, die von schwarz über braun bis hin zu goldgelb oder auch rot reichen. Des weiteren bietet jede Firma ihre eigene Farbausrichtung an, so daß beispielsweise der Farbton Mahagoni einer Firma anders wirkt als derselbe Farbton einer anderen Firma. Wie bereits erwähnt wird hierdurch versucht eine direkte Bindung des Kunden an den Hersteller einer entsprechenden Haartönung zu erzielen, da ein Wechseln des Herstellers und somit ein Verwenden eines anderen Produktes unweigerlich zu einem anderen Ergebnis führen würde.

Ein besonderer Nachteil der Haartönung liegt darin, daß sich graues Haar nur unzureichend abdecken läßt. Ein hierzu besser geeignetes Mittel sind die Haarfarben, die die Farbe der Kopfhaare beständig ändern. Insbesondere ist eine entsprechende, sog. permanente Haarfärbung, weitestgehend beständig gegen alle üblichen Haarbehandlungsmethoden sowie gegen Witterungseinflüsse und Licht. Somit wird ein Nachfärben der Haare nur aufgrund des Nachwachsens notwendig. Da jedoch bei der Anwendung von Haarfarben eine Reihe von Faktoren berücksichtigt werden müssen, um zu einem optimalen Ergebnis zu kommen, werden Haarfarben überwiegend vom professionellen Anwender, d.h. vom Frisör, angewendet.

Haarfarben werden zumeist in Form von Creme oder als Gel verarbeitet und sind üblicherweise in innenbeschichteten, spezial lackierten Aluminium-Tuben abgefüllt.

Auch in dem Bereich der Haarfarben hat jeder Hersteller seine eigene Farbabstimmung, wobei sich ungefähr fünfzig unterschiedliche Farbtöne in der Produktpalette der oben genannten Hersteller befinden. Dies führt nun zu einer großen Treue eines Frisörs zu seinem Hersteller, da der Frisör bei einem Wechsel des Herstellers seine Kundschaft an neue Produkte und Farben gewöhnen müßte oder diese gar verlieren könnte.

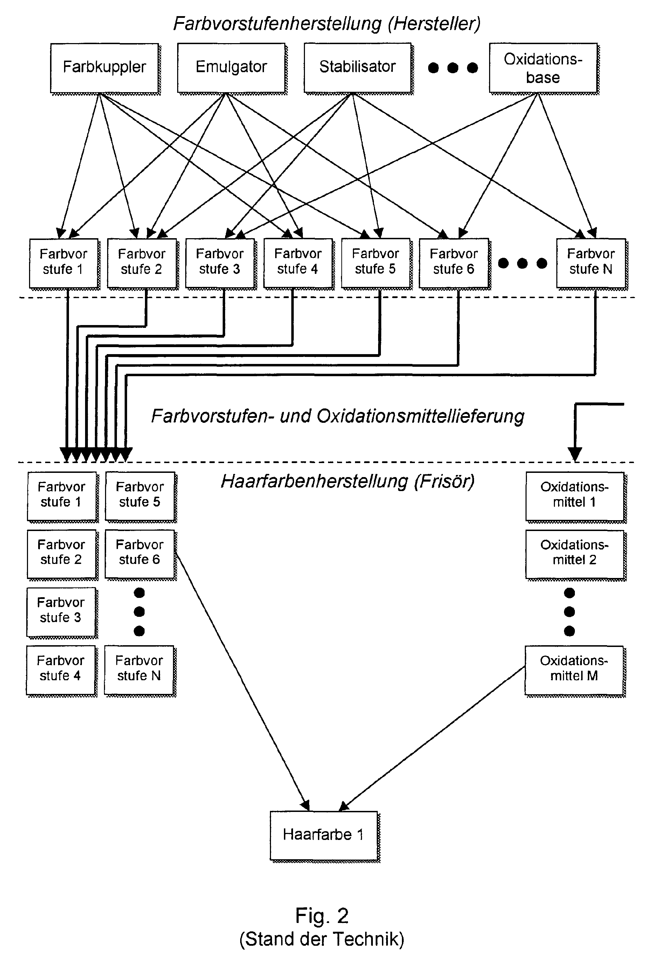

Die Herstellung von Haarfarben erfolgt gemäß dem Stand der Technik in einem zweistufigen Verfahren. In einer ersten Stufe werden von den Herstellern spezifische Grundkomponenten gemischt, um sog. Farbvorstufen herzustellen. In einer zweiten Stufe werden diese Farbvorstufen von einem Frisör durch Zumischen eines Oxidationsmittels oxidiert, wobei eine gebrauchsfertige Haarfarbe entsteht, die auf die Kopfhaare eines Kunden appliziert werden kann. Ein solches Herstellungsverfahren wird im folgenden anhand von Fig. 2 näher erläutert.

Fig. 2 illustriert beispielhaft eine gemäß dem Stand der Technik übliche Herstellung einer Haarfarbe.

In einer ersten Stufe erfolgt die Farbvorstufenherstellung, bei der von einem Hersteller Farbvorstufen hergestellt werden, durch die jeweils der entstehende Farbton der letztendlich hergestellten Haarfarbe weitgehend festgelegt wird. Ausgangspunkt der Herstellung sind Grundkomponenten, wie beispielsweise Farbkuppler, Emulgatoren, Stabilisatoren, Oxidations-Basen, oberflächenaktive Mittel, Mittel zur Viskositätseinstellung, Antioxidantien und Mittel zur Alkalitätseinstellung. Als Oxidations-Basen werden in der Regel leicht oxidierbare, aromatische Verbindungen verwendet, bevorzugterweise p-Phenylendiamin und p-Toluoldiamin (2,5 Diaminotoluolsulfat). Als Farbkuppler werden ebenfalls leicht oxidierbare aromatische Verbindungen verwendet, wobei vorzugsweise m-Phenylendiamin, m-Aminophenol, m-Dihydroxybenzol, Resorcin, 4-Chlorresorcin, 2-Methylresorcin, alpha Naphtol, Dihydroxynaphtalin, p-Nitro-o-phenylendiamine, 4-Amino-3-nitrophenol und 6-Chlor-4-nitro-2-aminophenol Anwendung finden. Als Alkalisierungsmittel wird überwiegend Ammoniak verwendet, jedoch auch organische Amine finden hier Anwendung. Als Penetrationsverstärker werden Harnstoff oder Alkohole, aber auch Glycole oder Glycolether verwendet.

Komplexierungsmittel (z. B. EDTA) und Stabilisierungsmittel (z. B. Salicylsäure), sowie Antioxidantien (z. B. Ascorbinsäure, Sulfite, Thioglycolsäure) werden ebenfalls benötigt. Des weiteren finden Duftstoffe und Emulgatoren, Tenside und Wachse, sowie Oele und quarternäre Pflegestoffe je nach Produktart, d. h. Shampoo oder Creme, einen breiten Anwendungsbereich.

In Abhängigkeit von dem zu erzielenden Farbton und den spezifischen, vorbestimmten Eigenschaften der herzustellenden Farbvorstufen werden einzelne Komponenten aus den oben aufgeführten Grundkomponenten ausgewählt. Somit können eine Vielzahl von Farbvorstufen, Farbvorstufe 1 bis Farbvorstufe N, hergestellt werden, die zur Färbung verschiedener Haartypen und Haarstrukturen verwendet werden können.

Ein professioneller Anwender von Haarfarben, d. h. ein Frisör, arbeitet nun in der Regel mit einem oder mehreren Herstellern dauerhaft zusammen, wobei der oder die Hersteller dem Frisör entsprechende Farbvorstufen, Farbvorstufe 1 bis Farbvorstufe N, zur Verarbeitung liefern. Zusätzlich muß der Frisör über Oxidationsmittel, Oxidationsmittel 1 bis Oxidationsmittel M, in ausreichender Menge verfügen, die er in der Regel ebenfalls von seinem Hersteller beziehen kann.

In einer zweiten Stufe kann der Frisör nun ausgehend von den Farbtönen und den jeweils entsprechenden Farbvorstufen, die von seinem oder seinen Hersteller(n) angeboten werden, für einen Kunden eine beschränkte Anzahl von Haarfarben herstellen. Dies erfolgt durch Zumischen eines bestimmen Oxidationsmittels zu der jeweiligen Farbvorstufe. Im vorliegenden Beispiel von Fig. 2 vermischt der Frisör Farbvorstufe 6 und Oxidationsmittel M zu Haarfarbe 1.

Ein besonderer Nachteil hierbei ist, daß mit keiner der angebotenen Farbvorstufen wirklich individuell gearbeitet werden kann und in der Regel immer Übermengen produziert werden, da die gebräuchlichen Farbvorstufen überwiegend hinsichtlich einer maximal benötigten Menge entsprechend vorherrschender Mengenstandards ausgeliefert werden. Insbesondere bedingt die Abhängigkeit von vorgefertigten Produkten, daß auch der Kundenwunsch sich häufig nicht vollständig erfüllen läßt, sowohl was den Farbton, als auch den Haartyp und die Haarstruktur betrifft. Um eine zufriedenstellende Kundenbetreuung zu gewährleisten, müßte jeder Frisör bis zu ca. 80 unterschiedlichen Farbvorstufen zuzüglich der unterschiedlichen Oxidationsmittel in ausreichendem Umfang ständig verfügbar halten. Des weiteren hängt der Preis einer Tönung oder Haarfarbe von der Menge der hergestellten Tönung bzw. Haarfarbe ab, d.h. der Kunde bezahlt zusätzlich evtl. produzierte Übermengen, die anschließend von dem Frisör entsorgt werden müssen.

Um dem Frisör ein größeres Farbsortiment zu ermöglichen, wurden Vorrichtungen zum maschinellen Mischen von Farbvorstufen und Oxidationsmitteln vorgeschlagen, wobei das Mischen auch rechnergesteuert erfolgen kann.

DE 41 13 454 A1 beschreibt eine Vorrichtung zum wahlweisen Dosieren von unterschiedlichen, fließfähigen Farbvorstufen, in der jeweils aus einem oder mehreren Behältern eine oder mehrere Farbvorstufen in einen Sammelbehälter abgegeben werden. Als Behälter können sowohl Beutel mit elastisch dehnbarem Innenbeutel und ggf. einem Produktabgabeventil, als auch Druckgasbehälter verwendet werden. Dies ermöglicht es, verschiedene Farbvorstufen vor Sauerstoff geschützt aufzubewahren und portionsweise zu entnehmen. Insbesondere kann einer der Behälter bereits ein Oxidationsmittel enthalten, das einer oder mehreren Farbvorstufen unmittelbar beigemischt werden kann zum Herstellen einer gebrauchsfertigen Haarfarbe. Dieses Herstellen der gebrauchsfertigen Haarfarbe kann insbesondere auch in Verbindung mit einem Rechner erfolgen, der eine jeweils aus einem Behälter zu entnehmende Produktmenge vorgibt und die entnommenen Produktmengen aufsummiert, so daß einem Anwender der Vorrichtung entsprechende Informationen über den Vorrat an einzelnen Farbvorstufen bereitgestellt werden. Des weiteren kann auch die Zugabe des Oxidationsmittels programmabhängig gesteuert werden, wobei für den Fall, daß zwei Behälter, die unterschiedliche Oxidationsmittelkonzentrationen enthalten, diese entsprechend von einem Frisör ausgewählt werden können. Somit ist die in der DE 41 13 454 A1 vorgeschlagene Vorrichtung unmittelbar zum Einsatz in Frisörsalons geeignet.

Obwohl die in DE 41 13 454 A1 vorgeschlagene Vorrichtung eine höhere Anzahl verwendbarer Farben ermöglicht, ist das zur Verfügung gestellte Farbspektrum bei vertretbarer Dimensionierung für den Vorort-Einsatz noch weit unter dem für eine optimale Kundenbetreuung notwendigen Maß.

Ausgehend von dem bekannten Stand der Technik und oben stehenden Nachteilen liegt der vorliegenden Erfindung die Aufgabe zugrunde, eine Möglichkeit zu schaffen, in effizienter Weise Haarfarben oder Tönungen vor Ort herzustellen, wobei ein großes Spektrum verschiedener Farben ermöglicht werden soll.

Diese Aufgabe wird durch die Gegenstände der Patentansprüche 1 und 12 gelöst. Bevorzugte Ausgestaltungen der Erfindung sind Gegenstand der Unteransprüche.

Insbesondere wird diese Aufgabe gelöst durch eine Verfahren und eine Vorrichtung zur maschinellen, individuellen Vorortherstellung von gebrauchsfertigen Haarfarben oder Tönungen. Hierbei erfolgt zuerst ein Bestimmen des von einem Kunden gewünschten Farbtons und der benötigten Farbmenge der herzustellenden Haarfarbe oder Tönung, ein separates Bereitstellen einer Vielzahl von für die Herstellung von Haarfarben oder Tönungen benötigten Farbkupplern, weiteren Grundkomponenten wie beispielsweise Oxidations-Basen, Emulgatoren, Alkalisierungsmitteln, Stabilisatoren und oberflächenaktiven Mitteln, sowie mindestens eines Oxidationsmittels und ein Bereitstellen von für unterschiedliche Haarfarben oder Tönungen benötigten Mischungsverhältnissen aus Farbkupplern, weiteren Grundkomponenten sowie Oxidationsmittel mittels eines Speichers. In Bezug auf die gewünschte Haarfarbe erfolgt nun ein Auslesen des korrespondierenden Mischungsverhältnisses aus dem Speicher, so daß ein Bestimmen der benötigten Dosierungsvolumina anhand des Mischungsverhältnisses und der benötigten Farbmenge erfolgen kann. Anschließend erfolgt ein maschinelles Zuführen und Vermischen der entsprechenden Dosierungsvolumina aus Farbkupplern, weiteren Grundkomponenten und Oxidationsmittel, so daß die benötigte hergestellte Menge der Haarfarbe oder Tönung ausgeben werden kann, wobei die Mange der Haarfarbe oder Tönung unmittelbar zur Applikation am Kunden verwendet werden kann.

Gemäß einer bevorzugten Ausführungsform der vorliegenden Erfindung wird eine Vorrichtung zur maschinellen, individuellen Vorortherstellung von gebrauchsfertigen Haarfarben oder Tönungen bereitgestellt, die im folgenden als "Color-Station" bezeichnet wird. Die Color-Station weist eine Vielzahl verschiedener Behälter auf, die jeweils Grundkomponenten, wie beispielsweise Farbkuppler, Emulgatoren, Stabilisatoren, Oxidations-Basen, oberflächenaktive Mittel, Mittel zur Viskositätseinstellung, Antioxidantien und Mittel zur Alkalitätseinstellung sowie Oxidationsmittel beinhalten. Des weiteren verfügt die Color-Station über einen Speicher zum Speichern verschiedener Mischungsverhältnisse, die die Zusammensetzung von unterschiedlichen Haarfarben oder Tönungen darstellen. Zum Herstellen einer benötigten Farbmenge einer Haarfarbe oder Tönung in einem von einem Kunden gewünschten Farbton können Informationen über eine Benutzer-Schnittstelle eingegeben werden. Hierbei werden nicht nur der gewünschte Farbton und die benötigte Farbmenge eingegeben, sondern vorzugsweise auch Informationen bezüglich der Haarstruktur des Kunden, beispielsweise dessen Eigenhaarfarbe, der Haarzustand, die Haarlänge, die vorherige Haarfarbe und/oder der Haarfarbwunsch sowie eine benötigte Alkalität und Viskosität der herzustellenden Haarfarbe oder Tönung. Im Anschluß hieran wird mittels einer Verarbeitungeinheit das zur gewünschten Haarfarbe korrespondierende Mischungsverhältnis aus dem Speicher ausgelesen, und die benötigten Dosierungsvolumina der einzelnen Grundkomponenten werden in bezug auf das Mischungsverhältnis und die benötigte Farbmenge bestimmt. Abschließend werden alle benötigten Dosierungsvolumina der Grundkomponenten und des Oxidationsmittels miteinander vermischt und ausgegeben, so daß der Frisör die hergestellte Farbmenge am Kopfhaar des Kunden applizieren kann.

Die Color-Station kann vorzugsweise zwischen 15 und 40 verschiedene Behälter aufnehmen, die vorzugsweise als Beutel mit einer Spezialkupplung ausgeführt sind, wobei die Spezialkupplung über einen Spezialmechanismus geöffnet wird. Hierdurch wird eine Entnahme einer bestimmten Menge aus einem entsprechenden Beutel ohne Sauerstoff-Berührung möglich. Zur Ausbringen einer Farbmenge aus einem Beutel werden Dosiereinrichtungen bereitgestellt, die vorzugsweise Spindeln mit Feingewinden aufweisen, so daß außerordentlich feine Dosierungen möglich sind.

Insbesondere erfolgt die Steuerung der Spindeln vorzugsweise über Elektromotoren, die genau steuerbare, programmvorgegebene Umdrehungen ausführen.

Ein besonderer Vorteil einer derartig ausgebildeten Color-Station besteht darin, daß der Frisör nahezu beliebig viele Permanent-Haarfarbtöne erstellen kann, die dem Kundenwunsch in optimaler Weise angenähert werden. Des weiteren können beliebige Farbtöne für Tönungen hergestellt werden, so daß auch hier der Frisör nahezu jedem Kundenwunsch gerecht werden kann.

Insbesondere ermöglicht die Color-Station dem Frisör, sich von marktführenden Herstellern zu lösen, ohne dadurch Risiken hinsichtlich des Verlustes seiner Kunden einzugehen. Zusätzlich können individuelle Farbmengen hergestellt werden, die einer tatsächlich benötigten Farbmenge entsprechen, so daß ein Herstellen von Übermengen im wesentlichen vermieden wird. Somit erlaubt die Color-Station ein umweltgerechtes Herstellen von Tönungen oder Haarfarben, da keine im Stand der Technik üblichen Übermengen mehr über den Ausguß entsorgt werden müssen. Des weiteren sind die Grundkomponenten-Beutel derart ausgeführt, daß die komplette Menge der jeweils enthaltenen Grundkomponente entnommen werden kann, so daß auch hier keine Restmengen entstehen, die entsorgt werden müssen.

Gemäß einer weiteren bevorzugten Ausführungsform der vorliegenden Erfindung wird die Color-Station in Verbindung mit einer graphischen Anzeige-Oberfläche bereitgestellt. Diese graphische Anzeige-Oberfläche ermöglicht das Anzeigen eines menschlichen Kopfes mit Kopfhaaren in Farbe und gleichzeitig die Farbanzeige von herstellbaren Haarfarben oder Tönungen, sowie verschiedenen Frisuren für eine Auswahl durch einen Kunden. Bei der graphischen Anzeige-Oberfläche handelt es sich vorzugsweise um einen sogenannten "Touchscreen", der auf Berührungen eines Benutzers reagiert und vorbestimmte Funktionen ausführt, so daß auf eine weitere Benutzer-Schnittstelle, wie beispielsweise eine Tastatur, verzichtet werden kann und der Platzbedarf somit minimiert wird. Zum Ausführen der bestimmten Funktionen werden eine Verarbeitungseinheit und ein Speicher bereitgestellt, in dem benötigte Informationen gespeichert werden.

Gemäß einem besonderen Aspekt der vorliegenden Erfindung wird in einer ersten Farbanzeige auf der Anzeige-Oberfläche ein menschlicher Kopf ohne Kopfhaar abgebildet. In derselben Anzeige wird eine Vielzahl verschiedener Frisuren im Kleinformat, d. h. beispielsweise in einer Sequenz von Kleinbildern, dargestellt, aus denen der Kunde durch Berühren des entsprechenden Kleinbildes auswählen kann. Hierbei können die verschiedenen Frisuren alles umfassen, was in gebräuchlichen Haarmodezeitschriften illustriert wird, d. h. von Kurz-Haar-Frisuren über Stufenschnitt-Frisuren bis hin zu Dauerwellen. Das obenstehende Berühren eines Kleinbildes bewirkt, daß die somit ausgewählte Frisur nun in der Anzeige als Kopfhaar des menschlichen Kopfes abgebildet wird. Auf diese Art kann der Kunde verschiedene Frisuren anzeigen lassen, um eine für sich selbst gewünschte Frisur zu bestimmen.

Anschließend kann der Kunde über eine entsprechende Berührung der graphischen Anzeige-Oberfläche zu einer Anzeige wechseln, in der der Kopf mit der ausgewählten Frisur und der Farbanzeige von herstellbaren Haarfarben oder Tönungen dargestellt wird. In dieser Anzeige werden vorzugsweise zuerst veschiedene Farbrichtungen angezeigt, ohne unmittelbar eine Vielzahl von Einzeltönen darzustellen, wobei die Einzeltöne bevorzugterweise in Farbgruppen, wie beispielsweise braun, schwarz, blond und farbig zusammengefaßt werden. Nun kann der Kunde über eine entsprechende Berührung einer angezeigten Gruppe von Farbtönen zu der Anzeige der möglichen Farben in bezug auf diese Gruppe wechseln. Berührt der Kunde anschließend eine bestimmte Farbe, so wird die derzeit angezeigte Farbe des Kopfhaares der ausgewählten Farbe angepaßt, so daß dem Kunden unmittelbar ein Eindruck vermittelt werden kann, wie die ausgewählte Frisur im Zusammenspiel mit der ausgewählten Haarfarbe sein Aussehen verändert würde. Nun kann der Kunde wiederum verschiedene Haarfarben auswählen, bis er die von Ihm gewünschte Haarfarbe bestimmt hat.

Des weiteren kann diese graphische Anzeige-Oberfläche derart ausgeführt werden, daß sie nur die Auswahl von Frisuren oder nur die Auswahl von Haarfarben oder Tönungen ermöglicht.

Gemäß einer weiteren bevorzugten Ausführungsform der vorliegenden Erfindung wird die Color-Station in Verbindung mit einer Benutzer-Schnittstelle und einer Schreib-/Leseeinheit bereitgestellt. Die Benutzer-Schnittstelle ermöglicht das Eingeben des von dem Kunden gewünschten Farbtons und der zur Erstellung der ausgewählten Frisur benötigten Farbmenge, sowie von Informationen bezüglich der Haarstruktur des Kunden, d. h. beispielsweise dessen Eigenhaarfarbe, der Haarzustand, die Haarlänge, die vorherige Haarfarbe und/oder der Haarfarbwunsch. Diese Daten werden nicht nur zum Auslesen eines entsprechenden Mischungsverhältnisses aus einem entsprechenden Speicher verwendet, sondern können vielmehr vorzugsweise mittels der Schreib-/Leseeinheit auf eine personalisierte Chipkarte des Kunden abgespeichert werden. Dies ermöglicht dem Kunden, jederzeit über Daten bezüglich durchgeführter Haarbehandlungen zu verfügen. Wenn beispielsweise Informationen hinsichtlich einer weit zurückliegenden Haarbehandlung, die den Kunden sehr zufriedengestellt hatte, benötigt werden, können diese problemlos aus der Chipkarte ausgelesen werden, so daß diese Haarbehandlung von dem Frisör unmittelbar reproduziert werden kann. Insbesondere kann auch die Zeit, die zwischen verschiedenen Haarbehandlungen verstreicht, Einfluß auf das bei einer erneuten Haarbehandlung anzuwendende Mischverhältnis haben, wobei dieser Einfluß in Abhängigkeit von aus der Chipkarte ausgelesenen Daten bestimmt werden kann.

Ein wesentlicher, weiterer Vorteil der vorliegenden Erfindung liegt daran, daß beim Herstellen einer Haarfarbe oder Tönung die benötigte Alkalität in bezug auf die Haarstruktur des Kunden individuell eingestellt werden kann. Somit kann die Haarfarbe oder Tönung optimal dem Kopfhaar des entsprechenden Kunden angepaßt werden.

Des weiteren besteht ein wesentlicher Vorteil der vorliegenden Erfindung darin, daß die Bestimmung der Mischungsverhältnisse programmgesteuert erfolgt. Dies ermöglicht dem Frisör ein sehr schnelles, effektives Bestimmen der benötigten Mischungsverhältnisse, da in dem Speicher der Color-Station eine sehr große Anzahl verschiedener Mischungsverhältnisse in bezug auf zur Verfügung stehender Grundkomponenten und möglicher Haarstrukturen gespeichert werden kann.

Insbesondere kann das erfindungsgemäße Vorortherstellen anstelle von Haarfarben oder Tönungen auch auf Shampoos, Festiger oder Dauerwellen-Lösungen angewendet werden.

Weitere bevorzugte Ausführungsformen der vorliegenden Erfindung werden anhand der folgenden Figuren näher erläutert. Die Figuren enthalten im einzelnen:
Fig. 1 ein Blockschaltbild zur Illustration einer Ausführungsform der erfindungsgemäßen, ein-stufigen Vorort-Haarfarbenherstellung; und
Fig. 2 ein Blockdiagramm zur Illustration einer Haarfarbenherstellung in einem zweistufigen Verfahren gemäß dem Stand der Technik.

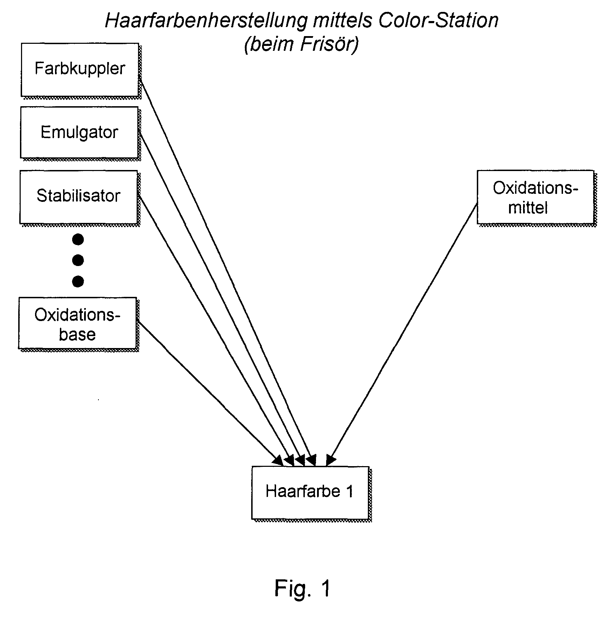

Fig. 1 ist ein Blockdiagramm zur Illustration eines ein-stufigen Herstellungsverfahrens zur individuellen Vorortherstellung von gebrauchsfertigen Haarfarben oder Tönungen, indem Grundkomponenten, wie Farbkuppler, Oxidations-Basen, Emulgatoren, Stabilisatoren, oberflächenaktive Mittel, Mittel zur Viskositätseinstellung, Antioxidantien, Lösungs- und Verdünnungsmittel, wie beispielsweise Wasser, Mittel zur Alkalitätseinstellung und jeweils genau definierte Mengen eines Oxidationsmittels in einem einzigen Arbeitsschritt zu einer unmittelbar gebrauchsfertigen Haarfarbe 1 vermischt werden.

Wie in Fig. 1 dargestellt ist, mischt die Color-Station für den Frisör unmittelbar alle für die Herstellung einer bestimmten Haarfarbe oder Tönung benötigten Grundkomponenten einschließlich des Oxidationsmittels. Hierbei ist gemäß einer bevorzugten Ausführungsform der vorliegenden Erfindung nur eine Oxidationsmittel-Konzentrat-Lösung notwendig, da zur Dosierung die benötigte Konzentration des Oxidationsmittels für eine Gesamtfarbmenge errechnet wird und nur eben diese Menge dosiert wird. Dies ermöglicht dem Frisör, eine beliebig große Anzahl verschiedener Farbtöne für Tönungen oder Haarfarben herzustellen. Im Stand der Technik wurden die besagten Grundkomponenten, d.h. alle oben aufgelisteten Komponenten mit Ausnahme des Wasserstoff-Peroxids, von einem Hersteller von Tönungen und Haarfarben in einem bestimmten Mischungsverhältnis gemischt, um Farbvorstufen herzustellen. Hierbei wird jedoch von jedem Hersteller aus marktstrategischen, kommerziellen Gründen nur ein Teil aller möglichen Mischungen dieser Grundkomponenten angeboten. Somit ergibt sich eine große Palette verschiedener Farbtöne, die nicht von dem Frisör genutzt werden können. Bei der Verwendung von Grundkomponenten hingegen ist es ausreichend, das Mischungsverhältnis dieser Grundkomponenten zu ändern, um zu einer anderen Tonhöhe zu gelangen, was untenstehend näher erläutert wird.

Gemäß einer weiteren Ausführungsform der vorliegenden Erfindung können gewisse Grundkomponenten zur Vereinfachung der Herstellung bzw. zur Vereinfachung der Lagerhaltung beim Frisör bereits vorab gemischt werden, ohne hierdurch die Vielfalt bei der Farbtonauswahl einzuschränken. Beispielsweise kann zu dem Oxidationsmittel bereits ein Stabilisator und Säure zugemischt werden, d. h. zu Wasserstoffperoxid wird vorab beispielsweise Salicylsäure und Phosphorsäure gemischt. Somit kann erreicht werden, daß eine geringere Anzahl von Behältern in einer entsprechenden Vorrichtung zur maschinellen, individuellen Vorortherstellung von gebrauchsfertigen Haarfarben oder Tönungen benötigt wird. So können beispielswiese Flüssighaarfarben bereits mit sieben verschiedenen Grundkomponenten erzielt werden.

Soll beispielsweise eine Flüssighaarfarbe mit einem gewünschten Farbton der Tonhöhe 7/0 und einer benötigten Farbmenge von 40 ml hergestellt werden, so kann dies mit nachstehend angegebenem Mischungsverhältnis erfolgen.

| | | |
|---|---|---|
| Beutel 1 | p-Phenylendiamin 1%ige wässrige Lsg. | 3,6 ml |
| Beutel 2 | m-Aminophenol, 1%ige wässrige Lsg. | 1,2 ml |
| Beutel 3 | Resorcinol 3%ige wässrige Lsg. | 4,6 ml |
| Beutel 4 | Demineralisiertes Wasser | 5,6 ml |
| Beutel 5 | Polyquaternium 11,15%ig | 0,2 ml |
| Beutel 6 | Ammoniak, 25%ige wässrite Lsg. | 1,2 ml |
| Beutel 7 | Wasserstoff-Peroxid 6%ige wässrige Lsg. | 23,6 ml |

Will man nun den Farbton ändern, beispielsweise auf eine Tönhöhe von 9/0, und wiederum eine Farbmenge von 40 ml Flüssighaarfarbe herstellen, so kann dies erzielt werden durch Ändern des Mischungsverhältnisses, unter Verwendung derselben Herstellungskomponenten:

| | | |
|---|---|---|
| Beutel 1 | p-Phenylendianmin 1 %ige wässrige Lsg. | 1,0 ml |
| Beutel 2 | m-Aminophenol, 1%ige wässrige Lsg. | 0,26 ml |
| Beutel 3 | Resorcinol 3%ige wässrige Lsg. | 1,32 ml |
| Beutel 4 | Demineralisiertes Wasser | 10,6 ml |
| Beutel 5 | Polyquaternium 11,15%ige Lsg. | 0,8 ml |
| Beutel 6 | Ammoniak, 25%ige wässrige Lsg. | 1,2 ml |
| Beutel 7 | Wasserstoff-Peroxid 6%ige wässrige Lsg. | 24,82 ml |

## Patentansprüche

1. Verfahren zur maschinellen, individuellen Vorortherstellung von gebrauchsfertigen Haarfarben oder Tönungen, wobei das Verfahren folgende Schritte aufweist:
Bestimmen des von einem Kunden gewünschten Farbtons und der benötigten Farbmenge der herzustellenden Haarfarbe oder Tönung;
separates Bereitstellen einer Vielzahl von für die Herstellung von Haarfarben oder Tönungen benötigten Farbkupplern, weiteren Grundkomponenten wie beispielsweise Oxidations-Basen, Alkalisierungsmitteln, Emulgatoren, Stabilisatoren und oberflächenaktiven Mitteln, sowie mindestens eines Oxidationsmittels;
Bereitstellen von für unterschiedliche Haarfarben oder Tönungen benötigten Mischungsverhältnissen aus Farbkupplern, weiteren Grundkomponenten sowie Oxidationsmittel mittels eines Speichers;
Auslesen des zur gewünschten Haarfarbe korrespondierenden Mischungsverhältnisses aus dem Speicher;
Bestimmen der benötigten Dosierungsvolumina anhand des Mischungsverhältnisses und der benötigten Farbmenge;
maschinelles Zuführen und Vermischen der entsprechenden Dosierungsvolumina aus Farbkupplern, weiteren Grundkomponenten und Oxidationsmittel, und
Ausgeben der benötigten hergestellten Menge der Haarfarbe oder Tönung zur Applikation am Kunden.

2. Verfahren nach Anspruch 1, **dadurch gekennzeichnet, daß** der Schritt des Bestimmens des gewünschten Farbtons das Bestimmen der Haarstruktur des Kunden umfaßt und **daß** die abgespeicherten Mischungsverhältnisse für unterschiedliche Haarstrukturen variieren.

3. Verfahren nach Anspruch 2, **dadurch gekennzeichnet, daß** das Bestimmen der Haarstruktur das Bestimmen von Eigenhaarfarbe, Haarzustand, Haarlänge, vorherige Haarfarbe und/oder Haarfarbwunsch umfassen.

4. Verfahren nach einem der Ansprüche 1 bis 3, **dadurch gekennzeichnet, daß** der Schritt des Bestimmens der benötigten Farbmenge das Bestimmen einer benötigten Alkalität der gewünschten Haarfarbe umfaßt und **daß** die abgespeicherten Mischungsverhältnisse für unterschiedliche Alkalitätseinstellungen variieren.

5. Verfahren nach Anspruch 4, **dadurch gekennzeichnet, daß** als Alkalisierungsmittel Ammoniak verwendet wird.

6. Verfahren nach einem der Ansprüche 1 bis 5, **dadurch gekennzeichnet, daß** der Schritt des Bestimmens der benötigten Farbmenge das Bestimmen einer benötigten Viskosität der gewünschten Haarfarbe umfaßt und **daß** die abgespeicherten Mischungsverhältnisse für unterschiedliche Viskositätseinstellungen variieren.

7. Verfahren nach einem der Ansprüche 1 bis 6, **dadurch gekennzeichnet, daß** neben Haarfarben oder Tönungen auch Shampoos, Festiger oder Dauerwellen-Lösungen in entsprechender Weise hergestellt werden.

8. Verfahren nach einem der Ansprüche 1 bis 7, **dadurch gekennzeichnet, daß** die Grundkomponenten die folgenden Rohstoffe umfassen: p-Phenylendiamin 0,01-1%ige Lösung, m-Aminophenol 0,01 - 1%ige Lösung, Resorcinol 0,01 - 4%ige Lösung, demineralisiertes Wasser, Ammoniak-Lösung 1 % - 25%ig, Wasserstoff-Peroxid 1% - 35%ig und 2,5 Diaminotoluolsulfat 0,01 - 1,5%ige Lösung.

9. Verfahren nach einem der Ansprüche 1 bis 8, **dadurch gekennzeichnet, daß** das separate Bereitstellen ein separates Bereitstellen von Teilabmischungen umfasst, wie beispielsweise Oxidationsmittel mit Stabilisatoren, oder Farbkuppler mit oberflächenaktivem Mitteln und/oder Antioxidantien und/oder geeigneten Lösungsmittel-Kombinationen und/oder Konservierungsmitteln.

10. Verfahren nach einem der Ansprüche 1 bis 9, **dadurch gekennzeichnet, daß** das Oxidationsmittel Wasserstoff-Peroxid oder Bromat ist.

11. Verfahren nach einem der Ansprüche 1 bis 10, **dadurch gekennzeichnet, daß** das Ausdosieren im wesentlichen Sauerstoff-frei erfolgt.

12. Vorrichtung zur maschinellen, individuellen Vorortherstellung von gebrauchsfertigen Haarfarben oder Tönungen, mit:
einer Benutzer-Schnittstelle zur Eingabe des von einem Kunden gewünschten Farbtons und der benötigten Farbmenge der herzustellenden Haarfarbe oder Tönung;
einer Vielzahl von Behältern jeweils enthaltend einen unterschiedlichen Farbkuppler;
einer Vielzahl von Behältern mit jeweils einer Grundkomponente wie beispielsweise einer Oxidations-Base, einem Emulgator, einem Stabilisator oder einem oberflächenaktiven Mittel;
mindestens einem Behälter mit Oxidationsmittel;
einem Speicher zum Speichern von für unterschiedliche Haarfarben oder Tönungen benötigten Mischungsverhältnissen aus Farbkupplern, weiteren Grundkomponenten sowie Oxidationsmittel;
einer Verarbeitungseinheit zum Bestimmen der benötigten Dosierungsvolumina bezüglich Farbkupplern, Grundkomponenten und Oxidationsmittel basierend auf der eingegebenen Farbtonwahl, Farbmenge und entsprechendem ausgegebenen Mischungsverhältnis;
einer Dosiereinheit zum maschinellen Zuführen und Vermischen der entsprechenden Dosierungsvolumina aus Farbkupplern, weiteren Grundkomponenten und Oxidationsmittel, und
einer Ausgabeeinheit zum Ausgeben der hergestellten Menge Haarfarbe oder Tönung zur Applikation am Kunden.

13. Vorrichtung nach Anspruch 12, **dadurch gekennzeichnet, daß** neben Haarfarben oder Tönungen auch Shampoos, Festiger oder Dauerwellen-Lösungen in entsprechender Weise hergestellt werden.

14. Vorrichtung nach Anspruch 12 oder 13, **dadurch gekennzeichnet, daß** die Behälter mit Farbkuppler jeweils einer der folgenden Rohstoffe enthalten: p-Phenylendiamin 0,01 - 1%ige Lösung, m-Aminophenol 0,01 - 1%ige Lösung, und Resorcinol 0,01 - 4%ige Lösung.

15. Vorrichtung nach einem der Ansprüche 12 bis 14, **dadurch gekennzeichnet, daß** die Behälter mit Grundkomponenten jeweils einen der folgenden Rohstoffe enthalten: demineralisiertes Wasser, Ammoniak-Lösung 1% - 25%ig, Wasserstoff-Peroxid 1% - 35%ig und 2,5 Diaminotoluolsulfat 0,01 - 1,5%ige Lösung.

16. Vorrichtung nach einem der Ansprüche 12 bis 15, **dadurch gekennzeichnet, daß** der mindestens eine Behälter mit Oxidationsmittel Wasserstoff-Peroxid 1%-35%ig enthält.

17. Vorrichtung nach Anspruch 12 oder 13, **dadurch gekennzeichnet, daß** die verschiedenen Behälter Teilabmischungen enthalten, wie beispielsweise Oxidationsmittel mit Stabilisator oder Farbkuppler mit oberflächenaktivem Mittel.

18. Vorrichtung nach einem der Ansprüche 12 bis 17, **dadurch gekennzeichnet, daß** der mindestens eine Behälter mit Oxidationsmittel Wasserstoff-Peroxid oder Bromat enthält.

19. Vorrichtung nach einem der Ansprüche 12 bis 18, **dadurch gekennzeichnet, daß** die Dosiereinheit im wesentlichen Sauerstoff-frei arbeitet.

20. Vorrichtung nach einem der Ansprüche 12 bis 19, **dadurch gekennzeichnet, daß** die Vorrichtung eine graphische Anzeige-Oberfläche aufweist.

21. Vorrichtung nach Anspruch 20, **dadurch gekennzeichnet, daß** die graphische Anzeige-Oberfläche ein Touchscreen ist, der auf Berührungen reagiert.

22. Vorrichtung nach einem der Ansprüche 12 bis 21, **dadurch gekennzeichnet, daß** die Vorrichtung eine Schreib- und Leseeinheit zum Schreiben von Daten bzw. Lesen der Daten in bzw. von einem Speicher einer Chipkarte, aufweist.
